# EUROPEAN PATENT APPLICATION

(11) **EP 0 856 327 A2**
(43) Date of publication of application: **05.08.1998**
(21) Application number: 98300073.8
(22) Date of filing: 07.01.1998
(51) Int. Cl.: A61M 16/10, A61M 39/26

(54) **Gas treatment devices and systems**

(30) Priority: 31.01.1997 GB 9701995
(71) Applicant: Smiths Industries Public Limited Company, London, NW11 8DS (GB)
(72) Inventor: Nash, John, Hythe, Kent, CT21 6QU (GB)
(74) Representative: Flint, Jonathan McNeill

(57) **Abstract**

An HME or filter has a gas-sampling port 20 on its casing 1 opening on one side of the HME element 10, 11 in the casing. The port 20 has a valve seat 27 and a spring-operated valve element 25, which normally closes the port but which is opened by insertion of a coupling 42 on the end of tubing 41 connected to a gas monitor 40, so that gas can flow to the monitor.

## Description

This invention relates to gas treatment devices of the kind having a treatment element within an outer casing, the casing having inlets on opposite sides of the element such that gas flows through the element, and a gas-sampling port.

Heat and moisture exchangers (HMEs) are used to warm and moisten gas supplied to a patient. The HME comprises a casing coupled in the patient breathing circuit, through which pass both inhaled and exhaled gas. An exchange element within the casing takes up a part of the heat and moisture in the expired gas and transfers a part of this to the inspired gas, when flow is reversed. The exchange element may be a coiled strip of corrugated paper treated with a hygroscopic material, or a foam. HMEs are sold by Portex Limited of Hythe, England under the trade mark Thermovent. Examples of HMEs are described in: GB 2277689; GB 2268496; GB2267840; EP 535016; EP 533644; EP 387220; EP 265163; EP 413127; US 4516573; US 4090513; US 4771770; US 4200094; and US 4048993. The HME may also include a filter for removing particles, bacteria and viruses from gas supplied to or from the patient.

HMEs often include a gas-sampling port to which can be attached a small bore tube extending to a gas monitor. When not in use, the port is closed by a removable cap. The need to remove this cap before access can be made to the gas-sampling port is a problem because there is a risk the cap may become lost after removal. If the cap should become lost, the HME may need to be replaced, which requires an interruption in the patient's ventilation. Also, it is generally desirable in surgical applications to reduce the number of small, loose items since these can present a hazard if they should inadvertently fall into a tube, wound site or the like. The problem can also exist with gas-sampling ports on, for example, filters.

It is an object of the present invention to provide an improved gas-treatment device and a system including a gas-treatment device and gas-sampling equipment.

According to one aspect of the present invention there is provided gas-treatment device of the above-specified kind, characterised in that the gas-sampling port includes a normally-closed valve, and that the valve is openable by connection of a coupling member to the port.

In this way, there is no need for any removable cap.

The treatment element may include a heat and moisture exchange element and, or alternatively, a filter. The port may include a tapered bore shaped to mate with a tapered surface on the coupling member.The port preferably includes a valve seat, the valve including a valve element having a radially-projecting flange and a spring arranged to urge the flange against the valve seat.

According to another aspect of the present invention there is provided a system including gas-sampling equipment and a gas-treatment device according to any one of the preceding claims, characterised in that the gas-sampling equipment includes a gas-sampling monitor, tubing extending from the monitor, and a coupling on an end of the tubing remote from the monitor, and that the coupling is engageable with the gas-sampling port such as to open the valve in the port and allow gas in the gas-treatment device to be supplied to the monitor.

An HME and gas-sampling system according to the present invention, will now be described, by way of example, with reference to the accompanying drawing, in which:

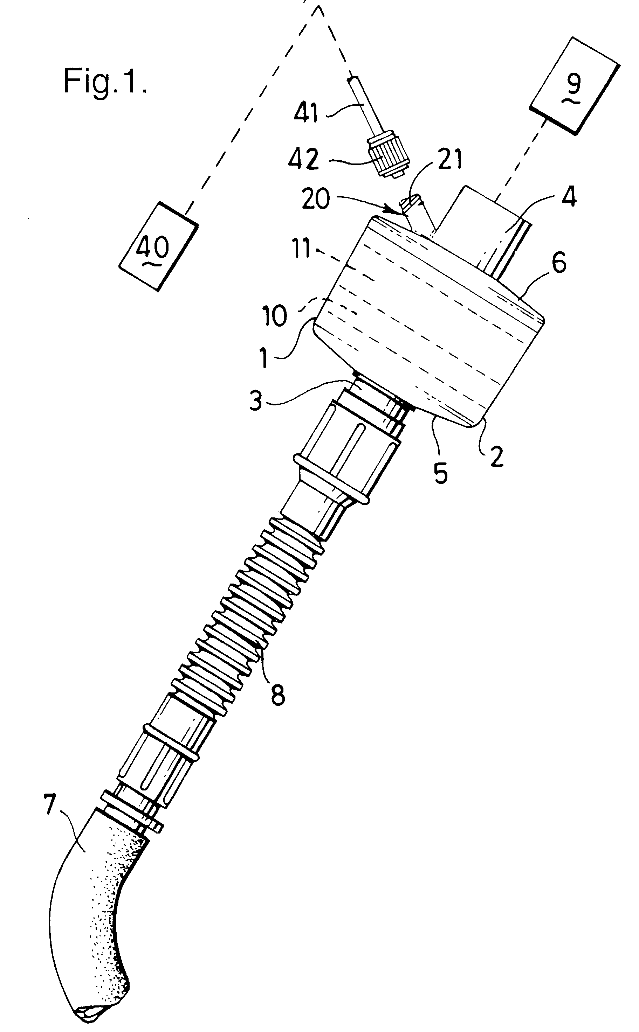

- Figure 1: is a side elevation view of a system including an HME and gas-sampling equipment; and
- Figure 2: is a sectional side elevation of the HME gas-sampling port to a larger scale before full connection of the gas-sampling coupling.

The HME has an outer casing 1 of generally cylindrical shape made from a rigid, transparent plastics material, such as polycarbonate. The casing 1 has an enlarged central region 2 2 between two inlets in the form of male and female tapered terminations 3 and 4 at opposite ends of the casing. The inlets 3 and 4 are connected with the central region 2 by shoulders 5 and 6 respectively. In use, the left-hand termination 3 is connected to a tracheal tube 7 (only a part of which is shown) via a corrugated, flexible catheter mount 8. Alternatively, the HME could be connected to the patient face mask or the like. The opposite termination 4 is connected to ventilation or anaesthetic equipment 9, in the usual way.

A conventional exchange element 10 is located within the central region 2 so that the inlets 3 and 4 are located at opposite ends of the element. The exchange element 10 may be a corrugated paper, foam or the like. The HME also includes a conventional bacterial and viral filter 11 located on the machine side of the HME element 10, that is, on the side opposite the patient. Alternatively, the element may be one member having both HME and filtering properties, or only HME or filtering properties.

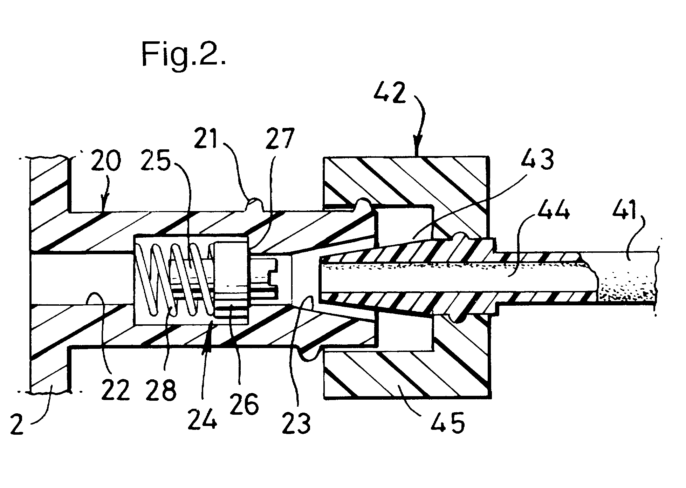

The casing 2 has a gas-sampling port 20 projecting from the shoulder 6 on the machine side of the HME element 10 and filter 11. The port 20 is a short cylindrical formation projecting outwardly at an angle of about 60° to the axis of the casing 2 and with a screw thread 21 around its external surface at its outer end. The port 20 is hollow with a bore 22 extending along its length. The outer end 23 of the bore 22 is tapered to a larger diameter, the inner end of the bore opening to the interior of the casing 2. The port contains a valve 24 comprising an axially-movable cylindrical valve element 25 with a radially-projecting flange 26. The valve element 25 is urged outwardly of the port 20 against an inwardly-facing annular valve seat 27 by a helical spring 28. In the natural position of the valve 24, as shown in Figure 2, with the valve element 25 in contact with the valve seat 27, flow of gas out of the port 20 is prevented. The diameter of the flange 26 is such as to allow free gas flow around its outside when displaced rearwardly off the valve seat.

The ventilation equipment 9 supplies gas to and from the patient via the filter 11 and HME element 10. Exhaled gas warms and moistens the HME element 10. Inhaled gas passing through the element 10 in the opposite direction takes up moisture and heat from the element so that the cold dry gas supplied to the ventilation equipment 9 is warmed and moistened before it is inhaled by the patient.

When it is desired to sample the respiratory gas, gas-monitoring equipment 40 is connected to the port 20 via small bore tubing 41. The port 20 is provided on the machine side of the HME so as to reduce the build-up of condensation at the monitor 40 and in the associated tubing 41. One end of the tubing 41 is connected to the monitor 40 whereas the other end is terminated by a conventional coupling 42 of the kind used on gas-sampling lines. The coupling 42 has a central nose 43 with a bore 44 extending along its length, which opens into the tubing 41. The nose 43 is tapered externally, such as with a luer taper, and its diameter and length are such that it can be inserted into the tapered end 23 of the bore 22 to make a friction, slip fit with the bore. In this position, the end of the nose 43 contacts the outer end of the valve element 25 and displaces it inwardly by a few millimetres, sufficient for it to clear the valve seat 27. Connection of the coupling 42, therefore, opens the valve 24 and allows gas in the HME casing 2 to escape through the port 20. The nose 43 forms a gas-tight seal in the bore 22 so that gas is confined to flow along the bore 44 of the nose into the tubing 41, and hence is supplied to the gas monitor equipment 40. The coupling 42 also has a rotatable locking ring 45, which is threaded internally to engage the external thread 21 on the port 20, so as to lock the coupling onto the port. The coupling may be of the kind described in GB2024974.

When gas sampling is no longer required, the coupling 42 is simply unscrewed and pulled out of the port 20, allowing the valve 24 to close, thereby preventing further escape of gas.

By including a valve in the gas-sampling port, there is no need for a separate removable component, such as a cap, to close the port after use. It can be seen that the port could have any conventional form of valve that is opened by connection of a coupling. For example, a duck-bill valve could be used, the nose of the coupling being inserted between the beaks of the valve.

## Claims

1. A gas-treatment device having a treatment element (10, 11) within an outer casing (1), the casing having inlets (3, 4) on opposite sides of the element (10, 11) such that gas flows through the element, and a gas-sampling port, characterised in that the gas-sampling port (20) includes a normally-closed valve (24), and that the valve (24) is openable by connection of a coupling member (42) to the port (20).

2. A gas-treatment device according to Claim 1, characterised in that the treatment element includes a heat and moisture exchange element (10).

3. A gas-treatment device according to Claim 1 or 2, characterised in that the treatment element includes a filter (11).

4. A gas-treatment device according to any one of the preceding claims, characterised in that the port (20) includes a tapered bore (23) shaped to mate with a tapered surface (43) on the coupling member (42).

5. A gas-treatment device according to any one of the preceding claims, characterised in that the port (20) includes a valve seat (27), and that the valve (24) includes a valve element (25) having a radially-projecting flange (26) and a spring (28) arranged to urge the flange (26) against the valve seat (27).

6. A system including gas-sampling equipment and a gas-treatment device according to any one of the preceding claims, characterised in that the gas-sampling equipment includes a gas-sampling monitor (40), tubing (41) extending from the monitor (40), and a coupling (42) on an end of the tubing (41) remote from the monitor (40), and that the coupling (42) is engageable with the gas-sampling port (20) such as to open the valve (24) in the port and allow gas in the gas-treatment device to be supplied to the monitor (40).
